# EUROPEAN PATENT APPLICATION

(11) **EP 3 195 785 A1**
(43) Date of publication of application: **26.07.2017**
(21) Application number: 15887720.9
(22) Date of filing: 02.10.2015
(51) Int. Cl.: A61B 1/00, G02B 23/24

(54) **CURVED RIGID ENDOSCOPE**

(30) Priority: 31.03.2015 JP 2015073084
(71) Applicant: Olympus Corporation, Tokyo 192-8507 (JP)
(72) Inventor: IMAI, Shigeru, Hachioji-shi Tokyo 192-8507 (JP)
(74) Representative: Schicker, Silvia
(86) International application number: PCT/JP2015/078061
(87) International publication number: WO 2016/157578

(57) **Abstract**

A bendable rigid endoscope 1 includes: a rigid insertion portion 2; a flexible bending portion 12; an operation portion 3 at which angle levers 14, 15 for bending the bending portion 12 are provided; and a finger rest/holding portion 17 including a finger rest portion 22 that allows a finger of a user inserted to the finger rest/holding portion 17 when the user holds the operation portion 3, the finger rest/holding portion 17 being provided on the insertion portion 2 side of the operation portion 3 relative to the angle lever 14, 15 in such a manner that the finger rest groove is pivotable around a longitudinal axis X of the operation portion 3.

## Description

### Technical Field

The present invention relates to a bendable rigid endoscope including a rigid insertion portion, such as those used in, e.g., a laparoscopic surgical operation.

### Background Art

In recent years, in the medical field and the industrial field, endoscope systems for observing the inside of a body cavity or the inside of an apparatus have widely been used.

In particular, in the medical field, various types of endoscopes such as bendable rigid endoscopes, which serve as surgical endoscopes suitable for use in observing, or performing treatment such as an operation of, the inside of an abdominal cavity by inserting an insertion portion from a hole poked in a body surface of, e.g., an abdominal part, as well as flexible endoscopes suitable for use in observing, or performing various treatment of, the inside of a digestive organ such as an esophagus, a stomach or a large bowel by inserting an insertion portion from an organ orifice such as a mouth cavity or an anus, have been in practical use.

Among these endoscopes, for example, as disclosed in Japanese Patent Application Laid-Open Publication No. 2012-45326, a bendable rigid endoscope used in an endoscopic surgical operation includes a rigid insertion portion, and an angle knob for bending a bending portion provided on the distal end side of the insertion portion is provided at an operation portion.

However, conventional bendable rigid endoscopes such as the one disclosed in Japanese Patent Application Laid-Open Publication No. 2012-45326, have a shape that allows anyone to easily grasp an operation portion, but face a need for improvement in holding capability when the operation portion is grasped in a predetermined position.

More specifically, for example, when a user wishes to grasp a bendable rigid endoscope used in laparoscopic surgery horizontally from a side, if the user grasps the operation portion so that the back of his/her hand faces upward to prevent the wrist of the user from being strained, large strain is put on the thumb that supports the weight of the abdominal endoscope, and thus, there has been a need for reduction in degree of fatigue during a long-time operation.

Therefore, the present invention has been made in view of the above problem, and an object of the present invention is to provide a bendable rigid endoscope that enables enhancement in capability of holding an operation portion and reduction in degree of fatigue of a user during a long-time operation.

### Disclosure of Invention

### Means for Solving the Problem

A bendable rigid endoscope according to an aspect of the present invention includes: a rigid insertion portion; a flexible bending portion provided at a distal end part of the insertion portion; an operation portion provided so as to be continuous with the insertion portion, an angle lever for bending the bending portion being provided at the operation portion; and a finger rest/holding portion including a finger rest groove that allows a finger of a user to be inserted to the finger rest groove when the user holds the operation portion, the finger rest/holding portion being provided on the insertion portion side of the operation portion relative to the angle lever in such a manner that the finger rest groove is pivotable around a longitudinal axis of the operation portion.

The above present invention enables provision of a bendable rigid endoscope that enables enhancement in capability of holding an operation portion and reduction in degree of fatigue of a user during a long-time operation.

### Brief Description of the Drawings

Fig. 1 is a perspective view illustrating an overall configuration of an endoscope apparatus according to an aspect of the present invention;
Fig. 2 is a side view of a configuration of the endoscope apparatus according to the aspect of the present invention;
Fig. 3 is a partial perspective view for describing fitting of a finger rest/holding portion to an operation portion, according to the aspect of the present invention;
Fig. 4 is a partial cross-sectional view of a distal end part of the operation portion at which the finger rest/holding portion is provided, according to the aspect of the present invention;
Fig. 5 is a side view illustrating a configuration of an endoscope apparatus according to a first modification of the aspect of the present invention;
Fig. 6 is a side view illustrating a configuration of an endoscope apparatus according to a second modification of the aspect of the present invention; and
Fig. 7 is a side view illustrating a configuration of an endoscope apparatus according to a third modification of the aspect of the present invention.

### Best Mode for Carrying Out the Invention

An endoscope apparatus, which is an abdominal endoscope, according to the present invention will be described below. Note that in the below description, the drawings based on the respective embodiments are schematic ones and, e.g., a relationship between a thickness and a width of each part and ratios in thickness among the respective parts are different from actual ones, and parts that are different in dimensional relationship and ratio depending on the drawings may be included in the drawings.

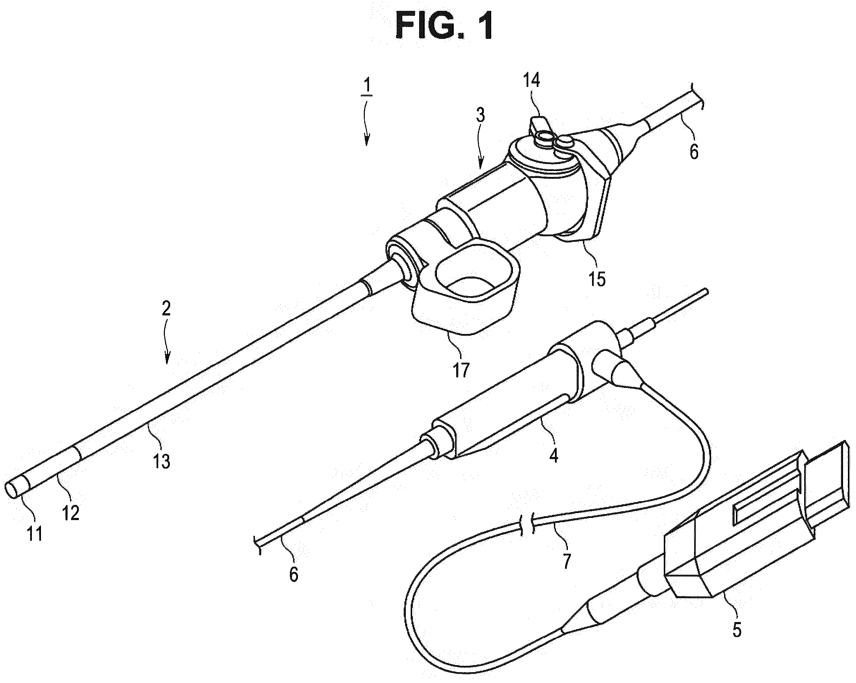

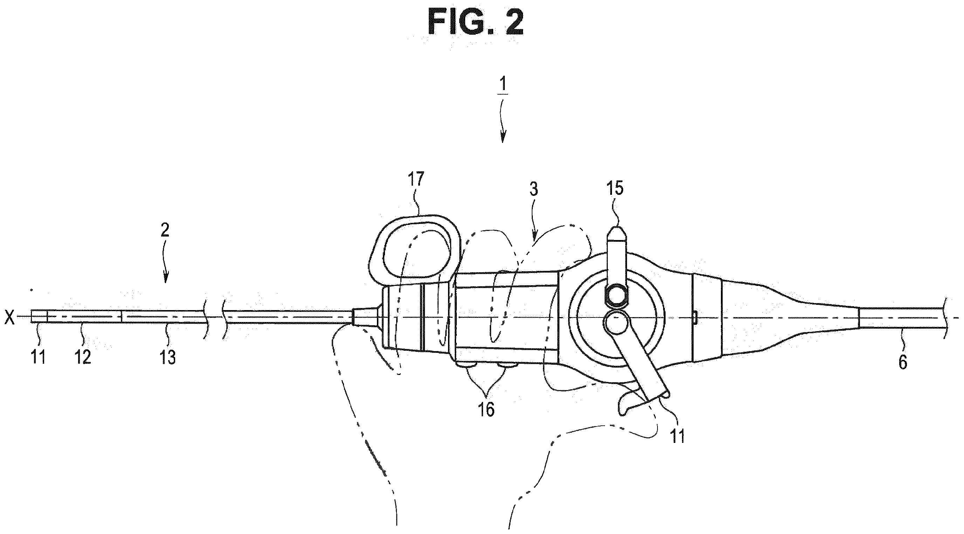

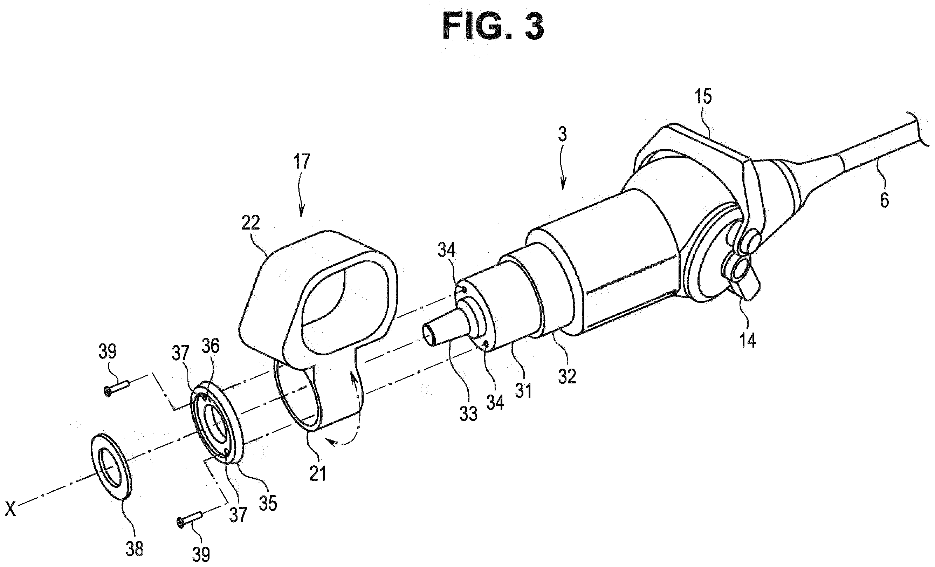

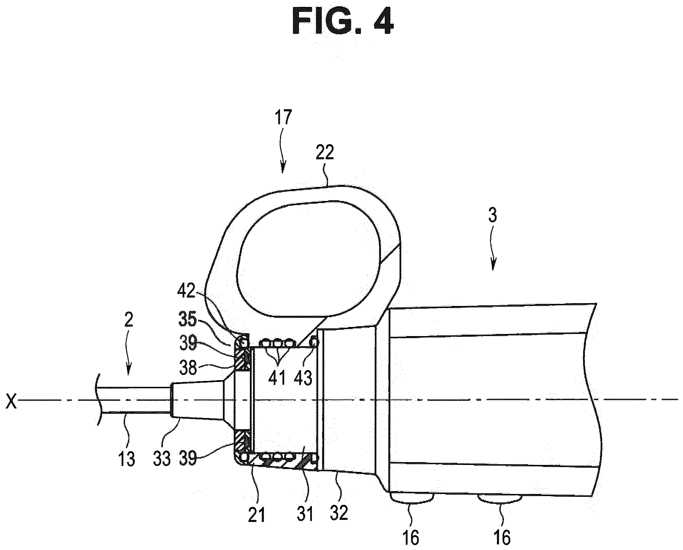

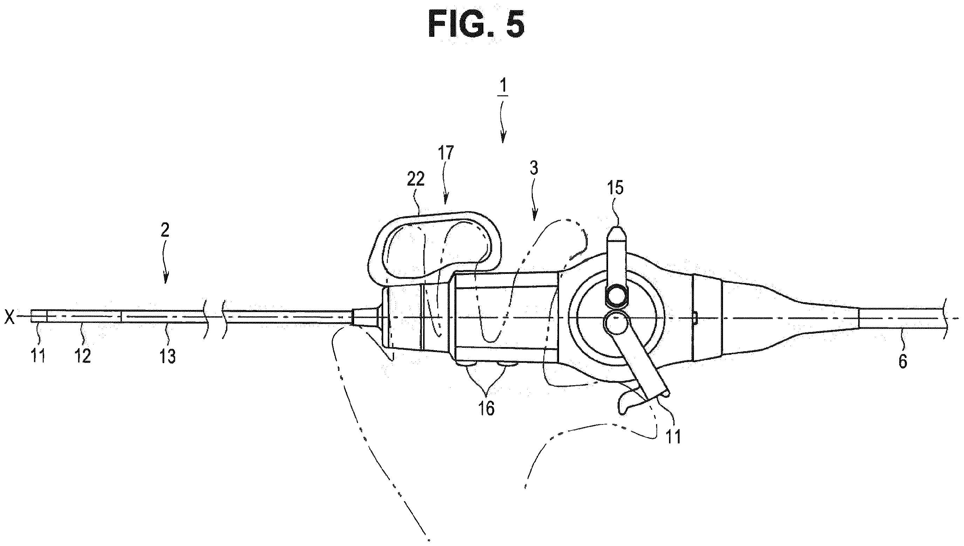

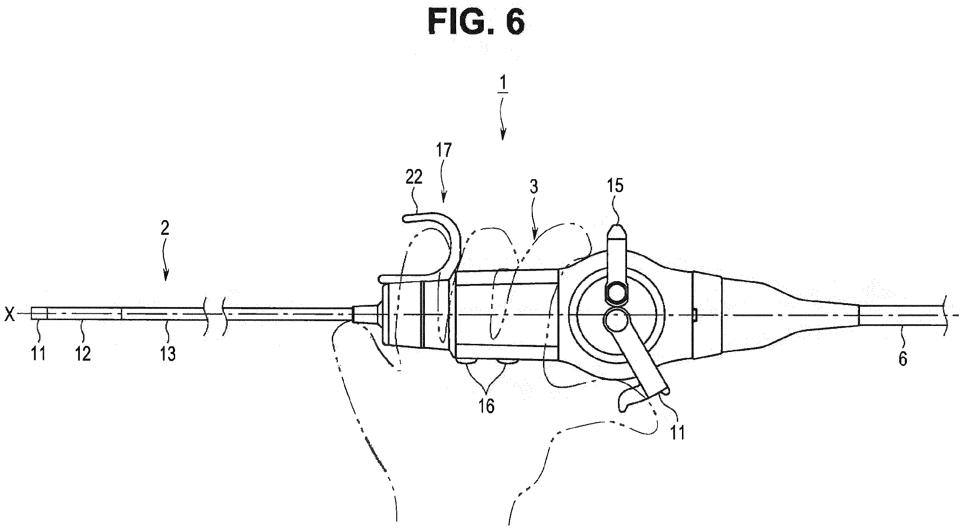

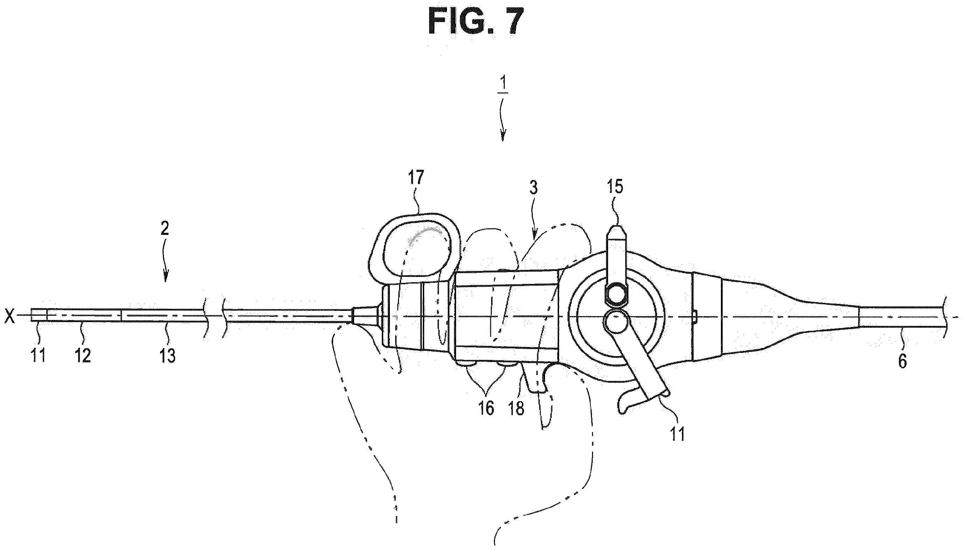

First, a bendable rigid endoscope according to an aspect of the present invention will be described with reference to the drawings. Fig. 1 is a perspective view illustrating an overall configuration of an endoscope apparatus that is a bendable rigid endoscope; Fig. 2 is a side view illustrating a configuration of the endoscope apparatus; Fig. 3 is a partial perspective view for describing fitting of a finger rest/holding portion to an operation portion; Fig. 4 is a partial cross-sectional view of a distal end part of the operation portion at which the finger rest/holding portion is provided; Fig. 5 is a side view illustrating a configuration of an endoscope apparatus according to a first modification; Fig. 6 is a side view illustrating a configuration of an endoscope apparatus according to a second modification; and Fig. 7 is a side view illustrating a configuration of an endoscope apparatus according to a third modification.

As illustrated in Fig. 1, an endoscope apparatus 1, which is a bendable rigid endoscope, mainly includes a long insertion portion 2, an operation portion 3 provided so as to be continuous with a proximal end of the insertion portion 2, a light guide connector 4 to be connected to a non-illustrated light source apparatus, and a video connector 5 to be connected to a non-illustrated video system center.

Here, in the endoscope apparatus 1, the operation portion 3 and the light guide connector 4 are connected via a universal cable 6, which is a flexible cable, and the light guide connector 4 and the video connector 5 are connected via a communication cable 7.

In the insertion portion 2, a distal end portion 11 mainly formed of a metal member of, e.g., stainless steel, a flexible bending portion 12 and a rigid tube 13 that is a metal tube of, e.g., stainless steel are provided continuously in this order from the distal end side. The insertion portion 2 is a part to be inserted into a body, and includes, e.g., non-illustrated cables and light guides incorporated inside.

The operation portion 3 includes angle levers 14, 15 for remotely operating the bending portion 12, various switches 16 (see Fig. 2) for operating, e.g., the light source apparatus or the video system center, and a finger rest/holding portion 17 that allows a finger of a user to be inserted and hooked in the finger rest/holding portion 17.

The angle levers 14, 15 provide bending operation means that enables the bending portion 12 of the insertion portion 2 to be operated in four, up/down and right/left, directions, that is, fully circumferentially.

Note that the bending portion 12 may be configured so as to bend only in two, up/down, directions. In such case, the angle lever 14 serves to remotely operate the bending portion 12, and the angle lever 15 serves as a fixing lever for fixing a bent state of the bending portion 12.

The above-described endoscope apparatus 1 according to the present embodiment is a bendable rigid endoscope that is what is a rigid endoscope used in a laparoscopic surgical operation, which includes an insertion portion 2, a large part other than the bending portion 12 of which is rigid.

Here, a configuration of the finger rest/holding portion 17 provided at the endoscope apparatus 1 according to the present embodiment will be described in detail below.

As illustrated in Fig. 3, the finger rest/holding portion 17 includes a finger rest portion 22 having a ring-like shape, here, which is a finger rest groove into which, for example, a ring finger of a user is inserted in such a manner as indicated by the alternate long and two short dashes line in Fig. 2, and an annular fitting portion 21 fitted to the operation portion 3 so as to be pivotable around a longitudinal axis X.

The finger rest/holding portion 17 is fitted by putting the fitting portion 21 on a pivot holding portion 31 provided at a distal end part of the operation portion 3, the pivot holding portion 31 providing a ring pivoting shaft and having an exact circle shape in cross-section.

Here, the fitting portion 21 of the finger rest/holding portion 17 is fitted from the distal end side of the pivot holding portion 31 until the fitting portion 21 abuts on a distal end face of a radially-projecting bulge portion 32 formed on the proximal end side of the pivot holding portion 31.

Then, a round plate ring-like retainer member 35 including a hole portion formed at a center is fixed to the pivot holding portion 31 via two screws 39, here, in such a manner that the round plate ring-like retainer member 35 abuts on a distal end face of the fitting portion 21. Note that, in the hole portion of the retainer member 35, a pipe sleeve portion 33 connected to the insertion portion 2 is inserted.

The retainer member 35 includes a recess portion 36 formed on the distal end side, and in the recess portion 36, insertion holes 37, through which the two screws 39 are inserted, respectively, are formed at two positions that are symmetrical with respect to a center point here.

The two screws 39 are threadably connected to respective screw holes 34 formed in a distal end face of the pivot holding portion 31, via the respective insertion holes 37 of the retainer member 35. Consequently, the retainer member 35 is fixed to the pivot holding portion 31.

Note that a round plate ring-like decorative cover 38 is bonded to the recess portion 36 of the retainer member 35 so as to prevent exposure of the screws 39. In this decorative cover 38, also, a hole portion, through which the pipe sleeve portion 33 connected to the insertion portion 2 is inserted, is formed at a center.

As described above, the distal end side of the fitting portion 21 of the finger rest/holding portion 17 abuts on the retainer member 35, and the proximal end side of the fitting portion 21 abuts on the bulge portion 32, and the fitting portion 21 is thus fitted to the distal end part of the operation portion 3 so as to be pivotable around the longitudinal axis X of the operation portion 3 and so as not to come off from the pivot holding portion 31.

The insertion portion 2 of the endoscope apparatus 1 configured as above is introduced into, for example, an abdominal cavity via, e.g., a trocar punctured and inserted in a patient during a laparoscopic surgical operation.

In this case, when a user grasps the endoscope apparatus 1 horizontally from a side, the user can grasp the operation portion 3 with a finger, for example, the ring finger of his/her hand inserted in the finger rest portion 22 of the finger rest/holding portion 17 as indicated by the alternate long and two short dashes line in Fig. 2.

Therefore, even if the user grasps the operation portion with the back of the hand facing upward, the insertion of the finger of his/her hand in the finger rest portion 22 of the finger rest/holding portion 17 reduces strain on the thumb supporting the weight of the endoscope apparatus 1 and thus reduces the degree of fatigue during long-time surgery.

In other words, even if the user opens the hand grasping the operation portion 3, the finger of his/her hand inserted in the finger rest portion 22 of the finger rest/holding portion 17 is caught, and thus, the position of the endoscope apparatus 1 is held without the endoscope apparatus 1 dropping down vertically.

Furthermore, the finger rest/holding portion 17 is pivotable around the longitudinal axis X of the operation portion 3 and thus can be moved to a desired pivotal position that allows the user to easily grasp the finger rest/holding portion 17.

According to the above, the endoscope apparatus 1 according to the present embodiment enables enhancement in capability of holding the operation portion 3 and significant reduction in degree of fatigue of a user during a long-time operation.

Note that, as illustrated in Fig. 4, on an inner circumferential face of the fitting portion 21 of the finger rest/holding portion 17, a plurality of, here, three O-shaped rings 41, which are resistive members that increase frictional resistance between the inner circumferential face of the fitting portion 21 and an outer circumferential face of the pivot holding portion 31 and adjust an amount of force required for movement around the longitudinal direction X of the operation portion 3 may be provided.

Consequently, the finger rest/holding portion 17 is less likely to be easily displaced from a desired pivotal position to which the finger rest/holding portion 17 has been moved by the user, enhancing a holding capability for maintaining the pivotal position.

Here, the resistive members are not limited to the O-shaped rings 41, and any of various components that provide frictional resistance enhancement such as a rubber tube may be employed.

Also, the inside of the fitting portion 21 of the finger rest/holding portion 17 is held in a water-tight manner even if the fitting portion 21 pivots, by an O-shaped ring 42, which is a water-tightness holding member on the retainer member 35, on the distal end side and an O-shaped ring 43, which is a water-tightness holding member abutting on the bulge portion 32, on the proximal end side.

Here, the water-tightness holding members are not limited to the O-shaped rings 42 and 43, and any of various seal structures may be employed.

Consequently, the endoscope apparatus 1 eliminates a need to remove the finger rest/holding portion 17 from the operation portion 3 for, e.g., cleaning, disinfection and/or sterilization before and after use and enables e.g., cleaning, disinfection and/or sterilization with the finger rest/holding portion 17 fitted to the operation portion 3.

### (First Modification)

As illustrated in Fig. 5, a finger rest portion 22 of a finger rest/holding portion 17 may have a horizontally-long shape widen along a longitudinal axis X of an operation portion 3 so as to allow a user to insert a plurality of fingers, here, two fingers, for example, a ring finger and a middle finger to the finger rest portion 22 as indicated in the alternate long and two short dashes line.

With such configuration, the endoscope apparatus 1 enables a position of the operation portion 3 to be more reliably held by insertion of two or more fingers to the finger rest portion 22 of the finger rest/holding portion 17 relative to insertion of one finger to the finger rest portion 22 of the finger rest/holding portion 17, without the operation portion 3 rotating (tilting) around the fingers, in addition to provision of various operation and effects described above.

### (Second Modification)

As illustrated in Fig. 6, a finger rest portion 22 of a finger rest/holding portion 17 is not limited to a ring-like shape and may have a horizontal U shape having a wall portion in the angle lever 14, 15 direction, which is the proximal end direction. The finger rest portion 22, which is a finger rest groove, here, is open on the distal end side so as to enable, for example, a ring finger to be easily introduced from the distal end side of the endoscope apparatus 1, which is different from a ring-like shape.

Here, the wall portion is provided on the proximal end side in the angle lever 14, 15 direction of the finger rest portion 22 so as to allow a ring finger to be caught and allow strength to be easily put in a thumb and an index finger operating the angle levers 14, 15, and ensure the capability of holding the operation portion 3 by holding the wall portion of the finger rest portion 22, for example, between the ring finger and the middle finger.

### (Third Modification)

As illustrated in Fig. 7, a hook portion 18 on which a thumb of a user is to be hooked and held may be provided between various switches 16 and an angle lever 14 of an operation portion 3 on the side substantially opposite to a position of a finger rest portion 22 of a finger rest/holding portion 17.

In other words, the hook portion 18 is provided on the side on which a finger, here, a thumb, of a user is positioned which holds and grasps the operation portion 3 with another finger, here, e.g., a ring finger, of the user which is inserted in the finger rest portion 22 of the finger rest/holding portion 17.

Consequently, the endoscope apparatus 1 provides a further enhanced holding capability by, for example, the ring finger of the user being inserted into the finger rest portion 22 of the holding portion 17 and the thumb of the user being hooked on the hook portion 18 so as to abut on the proximal end side of the hook portion 18.

The invention described in the above embodiment is not limited to the embodiment and the modifications of the embodiment, and in the practical phase, various other modifications are possible without departing from the spirit of the invention. Furthermore, the above-described embodiment includes various phases of the invention, and various aspects of the invention may be extracted by appropriate combinations of the plurality of elements disclosed.

For example, even where some elements are deleted from all the elements indicated in the embodiment, a configuration with such elements deleted may be extracted as an aspect of the invention if such configuration can solve a problem to be solved by the invention and provide an effect of the invention.

The present application is filed claiming priority from Japanese Patent Application No. 2015-073084 filed in Japan on March 31, 2015, the content of which is incorporated in the description, the claims and the drawings of the present application by reference.

## Claims

1. A bendable rigid endoscope comprising:
a rigid insertion portion;
a flexible bending portion provided at a distal end part of the insertion portion;
an operation portion provided so as to be continuous with the insertion portion, an angle lever for bending the bending portion being provided at the operation portion; and
a finger rest/holding portion including a finger rest groove that allows a finger of a user to be inserted to the finger rest groove when the user holds the operation portion, the finger rest/holding portion being provided on the insertion portion side of the operation portion relative to the angle lever in such a manner that the finger rest groove is pivotable around a longitudinal axis of the operation portion.

2. The bendable rigid endoscope according to claim 1, wherein a resistive member that adjusts an amount of force required for movement of the finger rest groove around the longitudinal axis is provided.

3. The bendable rigid endoscope according to claim 1 or 2, wherein a water-tightness holding member that holds the finger rest/holding portion and the operation portion in a water-tight manner is provided.

4. The bendable rigid endoscope according to any one of claims 1 to 3, wherein the finger rest portion is formed in a ring-like shape.

5. The bendable rigid endoscope according to claim 4, wherein the finger rest portion is formed in the ring-like shape that is horizontally long in the longitudinal axis direction of the operation portion so that a plurality of fingers of the user can be inserted to the finger rest portion.

6. The bendable rigid endoscope according to any one of claims 1 to 3, wherein the finger rest portion is formed in a horizontal U shape, a distal end side of the finger rest portion being open.

7. The bendable rigid endoscope according to any one of claims 1 to 6, comprising a hook portion provided at the operation portion, on a side on which a finger of the user is positioned, the finger holding and grasping the operation portion with another finger of the user, the other finger being inserted in the finger rest portion.
